# EUROPÄISCHE PATENTSCHRIFT

(11) **EP 3 825 685 B1**
(45) Veröffentlichungstag und Bekanntmachung des Hinweises auf die Patenterteilung: **21.09.2022**
(21) Anmeldenummer: 19000587.6
(22) Anmeldetag: 23.12.2019
(51) Int. Cl.: G01N 30/32, G01N 33/22

(54) **MOBILE MESSANLAGE ZUR DURCHFÜHRUNG EINER GASANALYSE**
MOBILE MEASURING SYSTEM FOR CARRYING OUT A GAS ANALYSIS
SYSTÈME DE MESURE MOBILE PERMETTANT DE RÉALISER UNE ANALYSE DE GAZ

(30) Priorität: 20.11.2019 DE 202019004724 U
(43) Veröffentlichungstag der Anmeldung: 26.05.2021
(73) Patentinhaber: Meter-Q Solutions GmbH, 35510 Butzbach (DE)
(72) Erfinder: ZACJ, Achim, 35510 Butzbach (DE); KÖRTE, Jost, 24857 Borgwedel (DE)

(56) Entgegenhaltungen:
- DE-U1-202017 005 215
- US-A1- 2013 192 339
- "Innovative Lösungen für die Bestimmung der Gasbeschaffenheit", GWF GAS+ ENERGIE, VULKAN VERLAG, Bd. 160, Nr. 11, 11. November 2019 (2019-11-11), Seiten 48-49, XP009521121, ISSN: 2366-9594

## Beschreibung

Die Erfindung betrifft eine mobile Messanlage zur Durchführung einer Gasanalyse, insbesondere zur Durchführung einer eichpflichtigen Gasanalyse, und / oder Bestimmung des Brennwertes und / oder Bestimmung des Wobbe-Indexes eines Prüfgases und / oder Bestimmung weiterer prozesstechnischer Daten zur Messung und / oder Überprüfung der eichpflichtigen Messdaten im Feld oder Labor.

Die europäische und damit auch die deutsche Erdgaswirtschaft befindet sich im Umbruch. Traditionelle Erdgasquellen wie aus den Niederlanden und der einheimischen deutschen Erdgasförderung nehmen jährlich ab. Das niederländische Erdgas wie das aus heimischer Produktion hat die sogenannte L-Gasqualität. Importe aus Quellen wie Russland, Norwegen, Dänemark oder anderer Länder haben H-Gas Qualität. Neue, regenerativ erzeugte Gase wie z.B. Biogas, synthetisches Methan oder auch Wasserstoff werden in die öffentlichen Gasversorgungsnetze eingespeist. Werden neue traditionelle Erdgasquellen erschlossen, geschieht das in der Regel außerhalb Europas. Zunehmend wird deshalb auch in Deutschland und Europa die Einspeisung von Flüssigerdgas (liquefied natural gas (LNG)) in das Erdgasnetz als Alternative zu existierenden Erdgasquellen vorgenommen, um hier auch der Abhängigkeit von Russland entgegenzuwirken.

Insbesondere die Einspeisung von regenerativ erzeugtem Wasserstoff ("blauer" oder "grüner" Wasserstoff) in das traditionelle Erdgasnetz mit einem Volumenanteil von bis zu 20% ist Teil der Strategie der Bundesregierung und der Erdgaswirtschaft im Rahmen der Energiewende. Um die eichpflichtig zu ermittelnden Brennwerte für die Abrechnung im Netz und an die Endverbraucher sicherzustellen, werden verstärkt sogenannte "Rekonstruktionssysteme" eingesetzt, die in Deutschland von der Physikalisch Technischen Bundesanstalt (PTB) für die Berechnung der Brennwerte in Erdgasnetzen zur eichpflichtigen Verrechnung zugelassen werden müssen. Um solche Rekonstruktionssysteme betreiben zu dürfen, schreibt die PTB in Abhängigkeit von der Netzstruktur vor, an bestimmten Stellen im Netz die Berechnungsergebnisse durch zeitweise, in der Regel 2- bis 4-wöchige Messungen pro Messstelle, Messungen zu überprüfen und zu validieren.

So konnte durch ein Projekt des Unternehmens E.ON SE gezeigt werden, dass in den letzten Jahren 30% der Haushaltskunden und 20% der Großkunden von starken Schwankungen des Wobbe-Index betroffen sind. Diese Schwankungen können bis zu maximal 1,5 kWh/m³ (ca. 15%) betragen. Die Variation der Gasbeschaffenheit und des verbundenen Brennwertes, und damit des gehandelten Energieinhaltes, in der Gaswirtschaft schlägt bis zum Endverbraucher durch.

Die Dynamik der Gasbeschaffenheit stellt die Energieabrechnung vor neue Herausforderungen und damit auch die eichpflichtige Messung der Gasbeschaffenheit und des abzurechnenden Brennwertes.

Die aktuell eingesetzten Messanlagen zur Bestimmung des Brennwertes und / oder des Wobbe-Index werden praktisch alle als stationäre Anlagen gebaut und eingesetzt. Seit einigen Jahren gibt es auch sogenannte "mobile PGC Anhänger-Anlagen", in denen eine konventionelle gaschromatographischen Anlage auf einen KFZ-Anhänger mit einem Gesamtgewicht von ca. 1.000 kg eingebaut wird.

Aus der US 2013/0192339 A1 ist eine Messanlage bekannt, die die Merkmale des Oberbegriffs des Patentanspruchs 1 aufweist.

Die DE 20 2017 005 215 U1 offenbart weiteren Stand der Technik.

Aus der Zeitschrift gwf Gas + Energie 11/2019, S. 48 - 49 - Produktvorschau zur gat 2019 ist eine mobile Messanlage zur Durchführung einer Gasanalyse bekannt. Die mobile Messanlage weist einen Gaschromatograph und ein elektrisch leitfähiges, transportfähiges Gehäuse auf, wobei der Gaschromatograph in dem Gehäuse eingebaut ist.

Aufgabe der vorliegenden Erfindung ist es, eine mobile Messanlage zu schaffen, die besonders platzsparend und einfach zu transportieren ist.

Gelöst wird diese Aufgabe durch eine mobile Messanlage, die die Merkmale des Schutzanspruchs 1 aufweist. Vorteilhafte Weiterbildungen der Messanlage sind Gegenstand der Unteransprüche.

Die erfindungsgemäße mobile Messanlage zur Durchführung einer Gasanalyse und / oder Bestimmung des Brennwertes und / oder Bestimmung des Wobbe-Indexes eines Prüfgases umfasst eine Messvorrichtung, wobei die Messvorrichtung einen Gaschromatograph, sowie eine Gasaufbereitungseinheit umfasst, wobei die Gasaufbereitungseinheit einen Druckregler, einen Durchflussregler, eine Filtereinheit sowie einen Bypass für das Prüfgas aufweist, wobei die mobilbe Messanlage ein elektrisch leitfähiges, transportfähigen Gehäuse aufweist, wobei die Messvorrichtung in dem Gehäuse eingebaut ist.

Eine Messanlage mit einem Gaschromatographen muss zur Messung eines in einer Pipeline geführten Gases an das Gasnetz über eine Gasentnahmestelle in der Pipeline angebunden werden. Mit Hilfe einer Entnahmesonde, die in der Pipeline eingebaut ist, gelangt das zu vermessende Gas über eine beheizte Probenleitung zur Messanlage. Der Druck des der Messanlage zugeführten, zu prüfenden/vermessenden Gases (Prüfgas) aus der Messschiene oder Pipeline ist normalerweise höher als der maximal zu verarbeitende Druck im Gaschromatograph der Messanlage. Daher muss der Druck auf den für den Gaschromatographen notwendigen Druck / Druckbereich reduziert werden. Danach folgend wird das Gas über einen Bypass zum Gaschromatographen geführt. Der Bypass kann auf eine höhere Durchflussrate eingestellt sein, als sie für den Gaschromatographen erforderlich ist, um zu jedem Zeitpunkt frisches, aktuelles Prüfgas zur Verfügung zu haben.

Mit der mobilen Messanlage wird erreicht, dass die Gasanalyse und / oder der Brennwert und / oder der Wobbe-Index schnell und einfach an dem entsprechenden Einsatzort durchgeführt werden kann, da die erfindungsgemäße mobile Messanlage sicher und leicht zu transportieren ist. So sind durch den Einbau der Messvorrichtung in das Gehäuse die empfindlichen Komponenten der Messanlage, insbesondere der Gaschromatograph und die Gasaufbereitungseinheit, besonders gut vor äußeren Einflüssen geschützt. Zudem erleichtert das Gehäuse den Transport der Messanlage an den Einsatzort. So kann die Messanlage einfach in normalen PKW an unterschiedlichen Messstellen gebracht werden um dort verwendet zu werden Vorzugsweise ist die Messvorrichtung derart gestaltet, dass die Gasanalyse und / oder die Ermittlung des Brennwerts und / oder des Wobbe-Indexex in einer Zeit <= 45 Sekunden erfolgt. Die Messanlage ist vorzugsweise derart gestaltet, dass sie in der Ex-Zone 1 oder 2 nach ATEX (ATEX 2017/34/EU Europäische Rcihtlinie zum Schutz von Anlagen ind explosionsgefährdeten Bereichen) oder IECEx (International Electrotechnical Commission System for Certification to Standards Relating to Equipment for Use in Explosive Atmospheres) installiert werden kann.

In einer bevorzugten Weiterbildung kann die Messanlage aufgrund des Gehäuses, insbesondere aufgrund des Gehäuses und der Anschlüsse, sowohl in geschlossenen Räumen als auch im Freien, vorzugsweise unter Einhaltung der eichpflichtigen Rahmenbedingungen, aufgestellt und betrieben werden.

Die mobile Messanlage gemäß Anspruch 1 ist geeignet zur Durchführung einer Gasanalyse und / oder Bestimmung des Brennwertes und/oder Bestimmung des Wobbe-Index eines Prüfgases. Die Messanlage umfasst eine Messvorrichtung, wobei die Messvorrichtung einen Gaschromatograph und eine Gasaufbereitungseinheit umfasst, wobei die Gasaufbereitungseinheit einen Druckregler, einen Durchflussregler, eine Filtereinheit sowie einen Bypass für das Prüfgas aufweist, wobei die mobile Messanlage ein elektrisch leitfähiges, transportfähiges Gehäuse aufweist, wobei die Messvorrichtung in dem Gehäuse eingebaut ist, wobei die Messanlage eine Montageplatte aufweist, wobei zumindest der Gaschromatograph und die Gasaufbereitungseinheit auf der Montageplatte montiert sind, insbesondere auch weitere Komponenten der Messvorrichtung auf der Montageplatte montiert sind.

Die Messanlage kann derart ausgebildet sein, dass die Gasaufbereitungseinheit einen weiteren Druckregler, einen weiteren Durchflussregler, eine weitere Filtereinheit sowie einen weiteren Bypass für ein Kalibriergas aufweist.

Die Messanlage kann derart ausgebildet sein, dass die Messanlage einen Prüfgasanschluss zur Anbindung an eine Probenentnahme aus einer Pipeline oder Prüfgasflasche aufweist, insbesondere der Prüfgasanschluss von außen zugänglich ist, vorzugsweise der Prüfgasanschluss an einer Außenseite des Gehäuses ausgebildet ist.

Die Messanlage kann derart ausgebildet sein, dass die Messanlage ein Kalibriergasanschluss aufweist, insbesondere der Kalibriergasanschluss von außen zugänglich ist, vorzugsweise der Kalibriergasanschluss an einer Außenseite des Gehäuses ausgebildet ist.

Die Messanlage kann derart ausgebildet sein, dass der Gaschromatograph mit Hilfe eines Kalibriergasanschlusses des Gaschromatographs und der Druckregelung, der Durchflussregelung und der Filterung für das Kalibriergas automatisch und/oder manuell kalibriert werden kann.

Die Messanlage kann derart ausgebildet sein, dass auch weitere Komponenten der Messvorrichtung auf der Montageplatte montiert sind.

Die Messanlage kann derart ausgebildet sein, dass die Montageplatte zur einfacheren Montage und Demontage mit Griffen versehen ist.

Die Messanlage kann derart ausgebildet sein, dass die Montageplatte, vorzugsweise die Montageplatte mit vormontierten Komponenten, insbesonder Komponenten der Messvorrichtung, mit lösbaren Verbindungen im Gehäuse befestigt werden kann und/oder ist. Die Messanlage kann derart ausgebildet sein, dass mit Hilfe des und/oder der Druchflussregler das Prüfgas und/oder das Kalibriergas zum Gaschromatographen über eine Zuleitung transportiert werden kann.

Die Messanlage kann derart ausgebildet sein, dass die Messanlage einen Trägergasbehälter aufweist, insbesondere einen als Trägergasflasche ausgebildeten Trägergasbehälter aufweist, wobei in dem Trägergasbehälter aufbewahrtes Trägergas über eine Zuleitung dem Gaschromatographen zugeführt werden kann.

Die Messanlage kann derart ausgebildet sein, dass der Trägergasbehälter und die Zuleitung in das Gehäuse eingebaut sind, vorzugsweise der Trägergasbehälter auf der Montageplatte montiert ist oder der Trägergasbehälter im Gehäuse hinter der Montageplatte montiert ist.

Die Messanlage kann derart ausgebildet sein, dass die der Messanlage zuführbahren Prüf- und/oder Kalibriergase aus den Bypassleitungen und das analysierte Gas aus dem Gaschromatographen einzeln oder über eine Ausblasesammelleitung aus dem Gehäuse herausgeführt und/oder einem oder mehreren Ausbläsern zugeführt wird.

Die Messanlage kann derart ausgebildet sein, dass die Messanlage eine Einrichtung zur Temperaturmessung und Datenspeicherung, insbesondere zur Überwachung, Aufzeichnung und Speicherung der innerhalb des Gehäuses herrschenden Umgebungstemperatur, vorzugsweise entsprechend des deutschen Eichgesetzes oder anderer normativer oder regulatorischer Anforderungen.

Die Messanlage kann derart ausgebildet sein, dass die Einrichtung in das Gehäuse eingebaut ist, vorzugsweise die Einrichtung auf der Montageplatte montiert ist.

Die Messanlage kann derart ausgebildet sein, dass die Messanlage eine elektronische Steuer- und Datenkommunikationseinheit zur Spannungsversorgung des Gaschromatographen und zur Datenübertragung vom und zum Gaschromatographen aufweist, insbesondere die elektronische Steuer- und Datenkommunikationseinheit zur Steuerungs- und/oder Abrechnungsdatenverbindung mittels eines Kabels mit dem Gaschromatographen verbunden ist.

Die Messanlage kann derart ausgebildet sein, dass die elektronische Steuer- und Datenkommunikationseinheit in das Gehäuse eingebaut ist, vorzugsweise die elektronische Steuer- und Datenkommunikationseinheit auf der Montageplatte montiert ist.

Die Messanlage kann derart ausgebildet sein, dass die Datenübertragung aus dem Gehäuse über die elektronische Steuerungs- und Kommunikationseinheit kabelgebunden und/oder kabellos erfolgen kann, insbesondere über Funk, WLAN oder ähnliches erfolgen kann.

Die Messanlage kann derart ausgebildet sein, dass das Gehäuse mit einem verschliessbaren Deckel ausgestattet ist.

Die Messanlage kann derart ausgebildet sein, dass der Deckel einen Verschluss aufweist und/oder mit einem Verschluss des Gehäuses verschließbar ist, vorzugsweise der Verschluss als Kniehebelverschluss oder Bügelverschluss ausgebildet ist.

Die Messanlage kann derart ausgebildet sein, dass der Deckel als klappbarer und/oder verschwenkbarer und/oder einhakbarer Deckel ausgebildet ist.

Die Messanlage kann derart ausgebildet sein, dass der Deckel mit Eichsiegeln versiegelt werden kann, so dass die Messanlage vor Ort von fachkundigem Personal, ohne sie zu öffnen und ohne Eichbeamten, in Betrieb genommen werden kann.

Die Messanlage kann derart ausgebildet sein, dass die mobile Messanlage einfach von maximal 2 Personen getragen und/oder gehandhabt werden kann.

Die Messanlage kann derart ausgebildet sein, dass die Messanlage ein Gesamtgewicht von unter 35 kg, insbesondere von unter 30 kg, vorzugsweise von unter 25 kg aufweist.

Die Messanlage kann derart ausgebildet sein, dass das Gehäuse Handgriffe, insbesondere ausklappbare Handgriffe, aufweist.

Die Messanlage kann derart ausgebildet sein, dass das Gehäuse Rollen aufweist, vorzugsweise Rollen und einen Griff aufweist, insbesondere Rollen und einen ausziehbaren Griff aufweist.

Die Messanlage kann derart ausgebildet sein, dass das Gehäuse als Tragekoffer mit Handgriffen oder als Rollkoffer mit Rollen zum einfacheren Rolltransport ausgebildet ist, insbesondere als Rollkoffer mit Rollen und mit einem Griff in fester oder ausziehbarer Form ausgebildet ist.

Die Messanlage kann derart ausgebildet sein, dass das Gehäuse einen Verschluss aufweist, wobei der Verschluss mit einem Siegel und/oder einer Sicherungsplombe gegen unerlaubtes Öffnen gesichert werden kann, insbesondere der Verschluss als Kniehebelverschluss oder Bügelverschluss ausgebildet ist.

Die Messanlage kann derart ausgebildet sein, dass die Messanlage einen Kalibriergasbehälter aufweist, wobei in dem Kalibriergasbehälter aufbewahrtes Kalibriergas über eine Zuleitung dem Gaschromatographen zugeführt werden kann.

Die Messanlage kann derart ausgebildet sein, dass der Kalibriergasbehälter und die Zuleitung in das Gehäuse eingebaut sind, vorzugsweise der Kalibriergasbehälter auf der Montageplatte montiert ist.

Nachfolgend wird bezugnehmend auf die Figuren die Erfindung näher erläutert ohne hierauf beschränkt zu sein.

Es zeigen:
- Figur 1: Schematische Darstellung einer Messanlage zur Gasanalyse und Ermittlung des Brennwertes und/oder des Wobbe-Index,

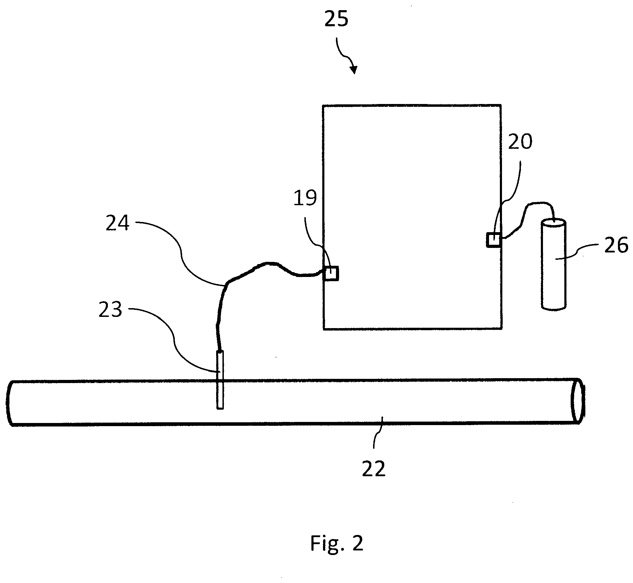

- Figur 2: Schematische Darstellung einer Anbindung der Messanlage an eine Pipeline und Probenentnahme mittels einer beheizten Probenleitung und an das Kalibriergas in einer druckbeaufschlagten Kalibriergasflasche,
- Figur 3: Mobile Messanlage mit einem als Rollkoffer ausgebildeten Gehehäuse.

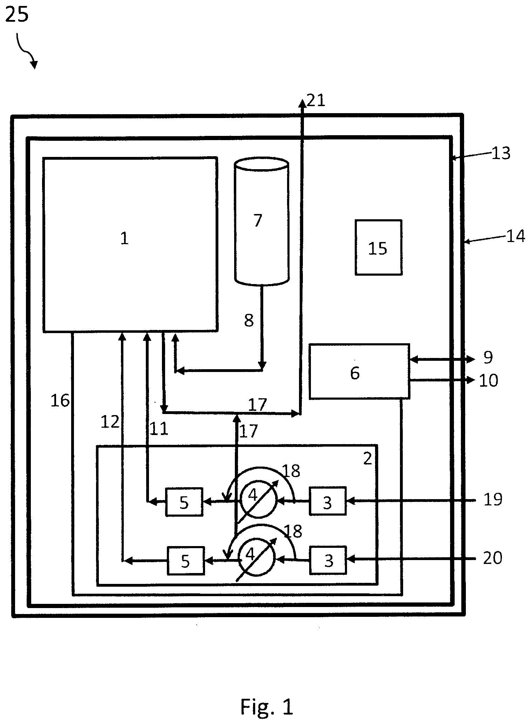

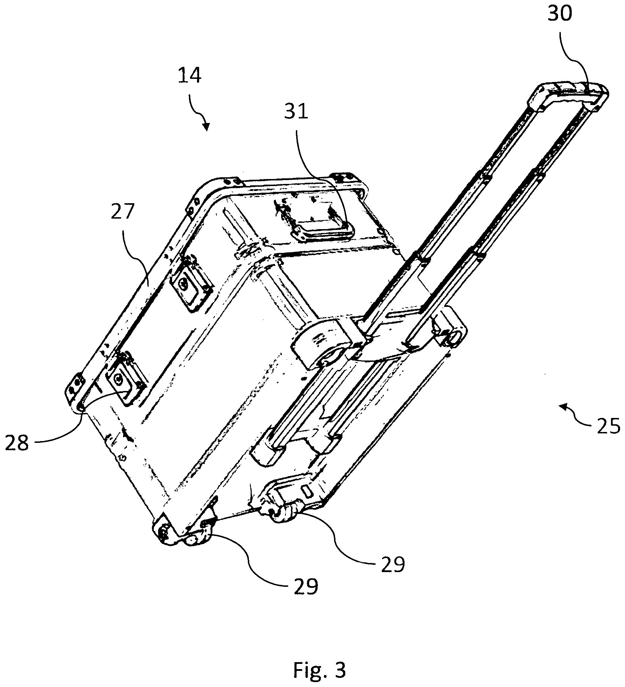

Die mobile Messanlage (25) zur Durchführung einer Gasanalyse und / oder Bestimmung des Brennwertes und / oder Bestimmung des Wobbe-Indexes eines Prüfgases umfasst eine Messvorrichtung, wobei die Messvorrichtung einen Gaschromatograph (1), sowie eine Gasaufbereitungseinheit (2) umfasst, wobei die Gasaufbereitungseinheit (2) einen Druckregler (3), einen Durchflussregler (4), eine Filtereinheit (5) sowie einen Bypass (18) für das Prüfgas aufweist. Ferner umfasst die Gasaufbereitungseinheit (2) einen weiteren Druckregler (3), einen weiteren Durchflussregler (4), eine weitere Filtereinheit (5) sowie einen weiteren Bypass (18) für ein Kalibriergas. Die mobilbe Messanlage (25) weist ein elektrisch leitfähiges, transportfähigen Gehäuse (14) auf, wobei die Messvorrichtung in dem Gehäuse (14) eingebaut ist. Dazu ist die gesamte Messvorrichtung auf einer Montageplatte (13) vormontiert. Die Montageplatte (13) wird dann in das elektrisch leitfähige, transportable Gehäuse (14) montiert. Dieses Gehäuse (14) kann aus praktischen Gründen als Koffer oder Rollkoffer mit Rollen (29), Handgriffen (31), ausziehbaren Zuggriff (30) und mit einem verschließbaren, abschließbaren Deckel (27) ausgebildet sein. Eine solche Ausführungsform zeigt die Figur 3. Das transportable Gehäuse (14) stellt neben der Funktion alsTransportbehältniseinen Schutz gegen mechanische und Umwelteinflüsse wie Staub, Spritzwasser, etc. dar. Auf der Montageplatte (13) ist der Gaschromatograph (1) montiert, der über druckfeste und gasdichte Zuleitung für das Prüfgas (11) und Zuleitung für das Kalibriergas (12) mit der Gasaufbereitungseinheit (2) verbunden ist. Die Gasaufbereitungseinheit (2) ist fest auf der Montageplatte (13) befestigt. In der Gasaufbereitungseinheit (2) sind die Druckregler (3), Durchflussregler (4), Bypassleitungen (18) und die Filtereinheit (5) mit Koaleszenzfilter und mechanischem Feinfilter mit der Zuleitung für Prüfgas (11) und der Zuleitung für das Kalibriergas (12) verbunden. Die Gasaufbereitungseinheit (2) ist über eine Zuleitung (19) mit einem Anschluss zum Zuführen des Prüfgases und einer Zuleitung (20) mit einem Anschluss zum Zuführen des Kalibriergases verbunden. Eine Flasche mit Trägergas (7) ist auf der Montageplatte (13) als auswechselbare Flasche montiert und mit einer druckfesten und gasdichten Rohrleitung (8) mit dem Gaschromatographen (1) verbunden. Die überschüssigen Gasmengen aus den Bypassleitungen (18) und das geprüfte Gas aus dem Gaschromatographen (1) werden über eine Rohrleitung einzeln oder wie bei der in der Figur 1 dargestellten Ausfürungsform als Ausblasesammelleitung (17) aus dem Innenraum des Gehäuses (14) nach außen an einen Ausbläser (21) geführt. Der Gaschromatograph (1) ist über Spannungsversorgungs- und Datenkommunikationskabel (16) mit der elektronischen Steuer- und Kommunikationseinheit (6) verbunden, die ebenfalls auf der Montageplatte (13) montiert ist. Diese elektronische Steuer- und Kommunikationseinheit (6) kann über eine Spannungsversorgung (9) und eine Datenkommunikationsleitung (10) über eine kabelgebundene Verbindung und/oder eine kabellose Verbindung, beispielsweise eine Funk- oder WLAN Verbindung, mit weiteren Systemen zur Steuerung und Datenübetragung verbunden werden. Außerdem kann die mobile Messanlage (25) eine ebenfalls mit der Montageplatte (13) verbundene, mit einer Batterie vorsorgte Einrichtung (15) aufweisen, die der Temperaturmessung und/oder Datenspeicherung dient.

Die mobile Messanlage (25) kann sowohl im permanenten, stationären Betrieb als auch in zeitlich begrenzten Anwendungsfällen z.B. für die zeitweise Messung in Rekonstruktionssystemen, als zeitweise Ersatzanlage bei Ausfällen von stationären Anlagen, während Instandhaltungsarbeiten oder Revisionen, etc. sehr schnell betriebsbereit eingesetzt werden. Die mobile Messanlage (25) kann vorab in einer staatlich anerkannten Prüfstelle oder Eichbehörde für den eichpflichtigen Verkehr kalibriert und mit einer Eichplombe versehen werden, so dass sie dann vor Ort ohne zusätzliche eichamtliche Kalibrierung sofort eingesetzt werden kann. Diese führt zu erheblichen Vereinfachungen in der Inbetriebnahme vor Ort und zu deutlichen Kosteneinsparungen.

Um ein in einer Pipeline (22) geführtes Gas, besipielsweise Erdgas, der mobilen Messanlage (25) als Prüfgas zuzuführen, kann eine in der Pipeline (22) gelagerte Entnahmesonde (23) mittels einer beheizten Probenleitung (24) mit der Zuleitung und dem Anschluss für das Prüfgas (19) verbunden werden. Zwecks Zuleitung von Kalibriergas zu der mobilen Messanlage (25) kann eine druckbeaufschlagte Kalibriergasflasche (26) mit der Zuleitung und dem Anschluss für das Kalibriergas (20) verbunden werden.

Insbesondere kann die Messanlage (25) aufgrund des Gehäuses (14), insbesondere aufgrund des Gehäuses (14) und der Anschlüsse (9), (10), (11), (12), (21), sowohl in geschlossenen Räumen als auch im Freien, vorzugsweise unter Einhaltung der eichpflichtigen Rahmenbedingungen, aufgestellt und betrieben werden kann.

### Bezeichnungsliste

- 1 -: Gaschromatograph
- 2 -: Gasaufbereitungseinheit
- 3 -: Druckregler
- 4 -: Durchflussregler
- 5 -: Filtereinheit mit Koaleszenz- und mechanischem Feinfilter
- 6 -: Elektronische Steuer und Kommunikationseinheit
- 7 -: Trägergasflasche
- 8 -: druckfeste und gasdichte Zuleitung für Kalibriergas
- 9 -: Spannungsversorgung
- 10 -: Datenkommunikationsleitung
- 11 -: druckfeste und gasdichte Zuleitung für Prüfgas
- 12 -: druckfeste und gasdichte Zuleitung für Kalibriergas
- 13 -: Montageplatte
- 14 -: Gehäuse
- 15 -: Einrichtung
- 16 -: Spannungsversorgungs- und Datenkommunikationskabel
- 17 -: Ausblasesammelleitung
- 18 -: Bypass
- 19 -: Zuleitung und Anschluss für Prüfgas
- 20 -: Zuleitung und Anschluss für Kalibriergas
- 21 -: Ausbläser
- 22 -: Erdgaspipeline
- 23 -: Entnahmesonde
- 24 -: beheizte Hochdruckleitung
- 25 -: mobile Messanlage
- 26 -: Kalibriergasflasche
- 27 -: Deckel
- 28 -: Verschluss
- 29 -: Rolle
- 30 -: Griff
- 31 -: Handgriff

## Patentansprüche

1. Mobile Messanlage (25) zur Durchführung einer Gasanalyse und / oder Bestimmung des Brennwertes und/oder Bestimmung des Wobbe-Index eines Prüfgases umfassend eine Messvorrichtung, wobei die Messvorrichtung einen Gaschromatograph (1) und eine Gasaufbereitungseinheit (2) umfasst, wobei die Gasaufbereitungseinheit (2) einen Druckregler (3) und einen Durchflussregler (4) aufweist, wobei die mobile Messanlage (25) ein transportfähiges Gehäuse (14) aufweist, wobei die Messvorrichtung in dem Gehäuse (14) eingebaut ist, **dadurch gekennzeichnet, dass** die Gasaufbereitungseinheit (2) eine Filtereinheit (5) sowie einen Bypass (18) für das Prüfgas aufweist, wobei der Durchflussregler (4) in Strömungsrichtung des Prüfgases hinter dem Druckregler (3) und vor der Filtereinheit (5) angeordnet ist, wobei der Bypass (18) zwischen dem Druckregler (3) und dem Durchflussregler (4) so angeordnet ist, dass überschüssige Gasmengen nach außen geführt werden, wobei das Gehäuse (14) ein elektrisch leitfähiges Gehäuse (14) ist, wobei die Messanlage (25) eine Montageplatte (13) aufweist, wobei zumindest der Gaschromatograph (1) und die Gasaufbereitungseinheit (2) auf der Montageplatte (13) montiert sind.

2. Messanlage nach Anspruch 1, wobei die Montageplatte (13) zur einfacheren Montage und Demontage mit Griffen versehen ist.

3. Messanlage nach Anspruch 1 oder 2, wobei die Montageplatte (13), vorzugsweise die Montageplatte (13) mit vormontierten Komponenten, mit lösbaren Verbindungen im Gehäuse (14) befestigt werden kann und/oder ist.

4. Messanlage nach einem der vorigen Ansprüche, wobei die Messanlage (25) einen Trägergasbehälter aufweist, insbesondere einen als Trägergasflasche (7) ausgebildeten Trägergasbehälter aufweist, wobei in dem Trägergasbehälter aufbewahrtes Trägergas über eine Zuleitung (8) dem Gaschromatographen (1) zugeführt werden kann.

5. Messanlage nach Anspruch 4, wobei der Trägergasbehälter und die Zuleitung (8) in das Gehäuse (14) eingebaut sind.

6. Messanlage nach Anspruch 5, wobei der Trägergasbehälter auf der Montageplatte (13) oder im Gehäuse (14) hinter der Montageplatte (13) montiert ist.

7. Messanlage nach einem der vorigen Ansprüche, wobei die Messanlage (25) eine Einrichtung (15) zur Temperaturmessung und Datenspeicherung, insbesondere zur Überwachung, Aufzeichnung und Speicherung der innerhalb des Gehäuses (14) herrschenden Umgebungstemperatur aufweist.

8. Messanlage nach Anspruch 7, wobei die Einrichtung (15) in das Gehäuse (14) eingebaut ist.

9. Messanlage nach Anspruch 8, wobei die Einrichtung (15) auf der Montageplatte (13) montiert ist

10. Messanlage nach einem der vorigen Ansprüche, wobei das Gehäuse (14) mit einem verschliessbaren Deckel (27) ausgestattet ist.

11. Messanlage nach Anspruch 10, wobei der Deckel (27) als klappbarer und/oder verschwenkbarer und/oder einhakbarer Deckel ausgebildet ist.

12. Messanlage nach einem der vorigen Ansprüche, wobei das Gehäuse (14) Rollen (29) aufweist, vorzugsweise Rollen (29) und einen Griff (30) aufweist, insbesondere Rollen (29) und einen ausziehbaren Griff (30) aufweist.

13. Messanlage nach einem der vorigen Ansprüche, wobei das Gehäuse (14) als Tragekoffer mit Handgriffen oder als Rollkoffer mit Rollen zum einfacheren Rolltransport ausgebildet ist, insbesondere als Rollkoffer mit Rollen und mit einem Griff in fester oder ausziehbarer Form ausgebildet ist.

14. Messanlage nach einem der vorigen Ansprüche, wobei die Gasaufbereitungseinheit (2) einen weiteren Druckregler (3), einen weiteren Durchflussregler (4), eine weitere Filtereinheit (5) sowie einen weiteren Bypass (18) für ein Kalibriergas aufweist.

15. Messanlage nach einem der vorigen Ansprüche, wobei die Messanlage (25) einen Kalibriergasbehälter aufweist, wobei in dem Kalibriergasbehälter aufbewahrtes Kalibriergas über eine Zuleitung dem Gaschromatographen (1) zugeführt werden kann, wobei der Kalibriergasbehälter und die Zuleitung in das Gehäuse (14) eingebaut sind, vorzugsweise der Kalibriergasbehälter auf der Montageplatte (13) montiert ist.

## Claims

1. Mobile measuring system (25) for carrying out a gas analysis and/or for determining the calorific value and/or for determining the Wobbe index of a test gas, comprising a measuring apparatus, wherein the measuring apparatus comprises a gas chromatograph (1) and a gas treatment unit (2), wherein the gas treatment unit (2) has a pressure regulator (3) and a throughflow regulator (4), wherein the mobile measuring system (25) has a transportable housing (14), wherein the measuring apparatus is fitted in the housing (14), **characterized in that** the gas treatment unit (2) has a filter unit (5) and a bypass (18) for the test gas, wherein the throughflow regulator (4) is arranged downstream of the pressure regulator (3) and upstream of the filter unit (5) in the flow direction of the test gas, wherein the bypass (18) is arranged between the pressure regulator (3) and the throughflow regulator (4) in such a way that surplus gas quantities are guided to the outside, wherein the housing (14) is an electrically conductive housing (14), wherein the measuring system (25) has a mounting plate (13), wherein at least the chromatograph (1) and the gas treatment unit (2) are mounted on the mounting plate (13) .

2. Measuring system according to Claim 1, wherein the mounting plate (13) is provided with handles for easier mounting and dismounting.

3. Measuring system according to Claim 1 or 2, wherein the mounting plate (13), preferably the mounting plate (13) with pre-mounted components, can be and/or is fastened in the housing (14) by way of releasable connections.

4. Measuring system according to one of the preceding claims, wherein the measuring system (25) has a carrier-gas container, in particular has a carrier-gas container in the form of a carrier-gas cylinder (7), wherein carrier gas stored in the carrier-gas container can be fed to the gas chromatograph (1) via a feed line (8).

5. Measuring system according to Claim 4, wherein the carrier-gas container and the feed line (8) are fitted in the housing (14).

6. Measuring system according to Claim 5, wherein the carrier-gas container is mounted on the mounting plate (13) or in the housing (14) behind the mounting plate (13).

7. Measuring system according to one of the preceding claims, wherein the measuring system (25) has a device (15) for temperature measurement and data storage, in particular for monitoring, recording and storage of the ambient temperature prevailing within the housing (14).

8. Measuring system according to Claim 7, wherein the device (15) is fitted in the housing (14).

9. Measuring system according to Claim 8, wherein the device (15) is mounted on the mounting plate (13).

10. Measuring system according to one of the preceding claims, wherein the housing (14) is equipped with a closable cover (27).

11. Measuring system according to Claim 10, wherein the cover (27) is in the form of a cover which can be folded and/or pivoted and/or hooked in.

12. Measuring system according to one of the preceding claims, wherein the housing (14) has wheels (29), preferably has wheels (29) and a handle (30), in particular has wheels (29) and an extendable handle (30) .

13. Measuring system according to one of the preceding claims, wherein the housing (14) is in the form of a carrying case with handles or in the form of a wheeled case with wheels for easier wheeled transport, in particular in the form of a wheeled case with wheels and with a handle in fixed or extendable form.

14. Measuring system according to one of the preceding claims, wherein the gas treatment unit (2) has a further pressure regulator (3), a further throughflow regulator (4), a further filter unit (5) and a further bypass (18) for a calibration gas.

15. Measuring system according to one of the preceding claims, wherein the measuring system (25) has a calibration-gas container, wherein calibration gas stored in the calibration-gas container can be fed to the gas chromatograph (1) via a feed line, wherein the calibration-gas container and the feed line are fitted in the housing (14), and the calibration-gas container is preferably mounted on the mounting plate (13).

## Revendications

1. Système de mesure mobile (25) destiné à effectuer une analyse de gaz et/ou déterminer le pouvoir calorifique et/ou déterminer l'indice de Wobbe d'un gaz test, ledit système de mesure comprenant un dispositif de mesure, le dispositif de mesure comprenant un chromatographe en phase gazeuse (1) et une unité de traitement de gaz (2), l'unité de traitement de gaz (2) comportant un régulateur de pression (3) et un régulateur de débit (4), le système de mesure mobile (25) comportant un boîtier transportable (14), le dispositif de mesure étant intégré dans le boîtier (14), **caractérisé en ce que** l'unité de traitement de gaz (2) comporte une unité de filtration (5) et une dérivation (18) destinées au gaz de test, le régulateur de débit (4) étant disposé en aval du régulateur de pression (3) et en amont de l'unité de filtration (5) dans le sens d'écoulement du gaz de test, la dérivation (18) étant disposée entre le régulateur de pression (3) et le régulateur de débit (4) de sorte que des quantités de gaz en excès soient dirigées vers l'extérieur, le boîtier (14) étant un boîtier électriquement conducteur (14), le système de mesure (25) comportant une plaque de montage (13), au moins le chromatographe en phase gazeuse (1) et l'unité de traitement de gaz (2) étant montés sur la plaque de montage (13).

2. Système de mesure selon la revendication 1, la plaque de montage (13) étant pourvue de poignées afin de faciliter le montage et le démontage.

3. Système de mesure selon la revendication 1 ou 2, la plaque de montage (13), de préférence la plaque de montage (13) pourvue de composants pré-assemblés, étant et/ou pouvant être fixée dans le boîtier (14) avec des liaisons amovibles.

4. Système de mesure selon l'une des revendications précédentes, le système de mesure (25) comportant un réservoir de gaz porteur, en particulier un réservoir de gaz porteur conçu sous la forme d'une bouteille de gaz porteur (7), le gaz porteur stocké dans le réservoir de gaz porteur pouvant être amené au chromatographe en phase gazeuse (1) par le biais d'une conduite d'alimentation (8) .

5. Système de mesure selon la revendication 4, le réservoir de gaz porteur et la conduite d'alimentation (8) étant intégrés dans le boîtier (14).

6. Système de mesure selon la revendication 5, le réservoir de gaz porteur étant monté sur la plaque de montage (13) ou dans le boîtier (14) derrière la plaque de montage (13).

7. Système de mesure selon l'une des revendications précédentes, le système de mesure (25) comportant un organe (15) de mesure de température et de stockage de données, en particulier de surveillance, d'enregistrement et de stockage de la température ambiante régnant à l'intérieur du boîtier (14).

8. Système de mesure selon la revendication 7, l'organe (15) étant intégré dans le boîtier (14).

9. Système de mesure selon la revendication 8, l'organe (15) étant monté sur la plaque de montage (13).

10. Système de mesure selon l'une des revendications précédentes, le boîtier (14) étant équipé d'un couvercle fermable (27).

11. Système de mesure selon la revendication 10, le couvercle (27) étant conçu comme un couvercle pliable et/ou pivotant et/ou accrochable.

12. Système de mesure selon l'une des revendications précédentes, le boîtier (14) comportant des rouleaux (29), de préférence des rouleaux (29) et une poignée (30), en particulier des rouleaux (29) et une poignée extensible (30).

13. Système de mesure selon l'une des revendications précédentes, le boîtier (14) étant conçu comme une mallette de transport pourvue de poignées ou comme une mallette roulante pourvue de roulettes pour faciliter le transport sur roulettes, en particulier comme une mallette roulante pourvue de roulettes et d'une poignée de forme fixe ou extensible.

14. Système de mesure selon l'une des revendications précédentes, l'unité de traitement de gaz (2) comportant un autre régulateur de pression (3), un autre régulateur de débit (4), une autre unité de filtration (5) et une autre dérivation (18) destinés à un gaz d'étalonnage.

15. Système de mesure selon l'une des revendications précédentes, le système de mesure (25) comportant un réservoir de gaz d'étalonnage, le gaz d'étalonnage stocké dans le réservoir de gaz d'étalonnage pouvant être amené au chromatographe en phase gazeuse (1) par le biais d'une conduite d'alimentation, le réservoir de gaz d'étalonnage et la conduite d'alimentation étant intégrés dans le boîtier (14), de préférence le réservoir de gaz d'étalonnage étant monté sur la plaque de montage (13).
